# EUROPÄISCHE PATENTSCHRIFT

(11) **EP 2 158 371 B1**
(45) Veröffentlichungstag und Bekanntmachung des Hinweises auf die Patenterteilung: **03.04.2013**
(21) Anmeldenummer: 08801446.9
(22) Anmeldetag: 04.06.2008
(51) Int. Cl.: E05D 3/06, E05D 11/00, F25D 23/02

(54) **KÜHL- UND/ODER GEFRIERGERÄT**
COOLING AND/OR FREEZING UNIT
APPAREIL DE RÉFRIGÉRATION ET/OU DE CONGÉLATION

(30) Priorität: 09.06.2007 DE 202007008106 U
(43) Veröffentlichungstag der Anmeldung: 03.03.2010
(73) Patentinhaber: Liebherr-Hausgeräte Lienz GmbH, 9900 Lienz (AT)
(72) Erfinder: OBERHAUSER, Florian, 8010 Graz (AT); KÖFELE, Markus, A-9961 Hopfgarten (AT)
(74) Vertreter: Herrmann, Uwe
(86) Internationale Anmeldenummer: PCT/EP2008/004453
(87) Internationale Veröffentlichungsnummer: WO 2008/151747

(56) Entgegenhaltungen:
- EP-A- 1 191 289
- DE-A1- 3 510 670
- DE-A1- 4 122 541
- GB-A- 1 096 733

## Beschreibung

Die vorliegende Erfindung betrifft eine Anordnung zur Führung eines Kabels von einem Korpus eines Möbelelementes oder Gerätes zu einem Verschlußelement, das relativ zu dem Korpus mittels eines Scharniers verschwenkbar ist und mittels dessen der Korpus verschließbar ist.

Bei zahlreichen Haushaltsgeräten, wie beispielsweise bei Kühl- und/oder Gefriergeräten besteht häufig der Bedarf des Einsatzes von Displays in der Tür oder Klappe. Um derartige Displays mit Strom und/oder Daten zu versorgen, ist es erforderlich ein oder mehrere Kabel von dem Korpus des Gerätes in die Tür oder Klappe zu legen.

Die EP 1 191 289 A2 betrifft ein Türscharnier für ein Küchengerät, bei dem ein Kabel durch einen ersten Teil des Türscharniers durch eine im ersten Teil des Türscharniers angebrachte längliche, tunnelartige Öffnung geführt wird und wobei das Kabel auf einem zweiten Teil des Türscharniers mittels eines gefederten Halteteils gehalten wird.

Die DE 41 22 541 A1 betrifft ein Scharnier für Kraftfahrzeugtüren, wobei die Scharnierteile als kreisbogenförmige, im Querschnitt U-förmige, ineinander gleitend ausgelegte, offene Kanäle ausgebildet sind, in denen ein Kabel geführt ist.

Die DE 3510 670 A1 offenbart ein Scharnier mit allen Merkmalen des Oberbegriffs des Anspruchs 1.

Der vorliegenden Erfindung liegt die Erfindung zugrunde, eine Anordnung der eingangs genannten Art bereitzustellen, bei der das oder die Kabel zahlreichen Öffnungs- und Schließbewegungen der Tür oder Klappe standhält und somit insbesondere die Wahrscheinlichkeit für das Auftreten von Ermüdungsbrüchen verringert ist.

Diese Aufgabe wird durch eine Anordnung mit den Merkmalen des Anspruchs 1 gelöst. Danach ist vorgesehen, dass die Anordnung wenigstens ein erstes Halteteil und wenigstens ein zweites Halteteil zur Führung des Kabels aufweist, wobei das erste Halteteil an einem ersten Teil des Scharniers und wobei das zweite Halteteil an einem zweiten Teil des Scharniers angeordnet ist, das relativ zu dem ersten Teil des Scharniers bewegbar ist. Die Halteteile sind dabei derart ausgeführt, dass sie das Kabel derart führen, dass eine Öffnungs- und Schließbewegung des Verschlußelementes möglich ist, ohne dass diese durch das Kabel behindert wird und ohne dass das Kabel dabei starken Belastungen ausgesetzt ist. Vorzugsweise ist vorgesehen, dass die Halteteile derart angeordnet sind, dass das Kabel bei der Öffnungs- oder Schließbewegung des Scharniers nur elastischen Verformungen ausgesetzt ist, was Ermüdungsbrüche verhindert oder zumindest die Wahrschein-lichkeit für deren Auftreten verringert. Die Halteteile weisen erfindungsgemäß Nuten oder Klammern oder dergleichen auf, mittels derer das Kabel geführt wird.

Erfindungsgemäß weist das Scharnier einen Korpusbeschlagskörper und einen Verschlusselementbeschlagsköper sowie eine oder mehrere zwischen bindungselemente auf, wobei das erste und/oder das zweite Teil des Scharniers durch das wenigstens eine Verbindugselement gebildet wird. Diese

Das erste und/oder das zweite Halteteil können mit dem ersten bzw. zweiten Teil des Scharniers verschraubt oder durch eine Steckverbindung fixiert sein.

Denkbar ist es, dass das erste bzw. das zweite Teil des Scharniers durch den Korpusbeschlagskörper und/oder den Verschlusselementbeschlagskörper gebildet wird. Möglich ist es beispielsweise, dass das zweite Halteteil durch eine Steckverbindung mit dem Verschlusselementbeschlagskörper in Verbindung steht, d.h. mit dem Beschlagskörper, der im montierten Zustand des Scharniers mit dem Verschlußelement, d.h. z.B. mit der Tür oder Klappe in Verbindung steht.

Verbindungselemente können als Hebel ausgeführt sein, die miteinander und/oder mit dem Korpusbeschlagskörper und/oder mit dem Verschlusselementbeschlagskörper schwenkbar in Verbindung stehen. Denkbar ist es beispielsweise, dass das erste Halteteil mit einem dieser Verbindungselemente durch eine Schraubverbindung in Verbindung steht.

In weiterer Ausgestaltung der Erfindung kann vorgesehen sein, dass das erste Halteteil sowie das zweite Halteteil Kabelführungsbereiche aufweisen, die wenigstens abschnittsweise auf gleicher Höhe angeordnet sind. Diese Ausführung der Erfindung ermöglicht es, die Abschnitte des Kabels zwischen dem ersten und dem zweiten Halteteil möglichst kurz auszugestalten.

Das erste Halteteil und/oder das zweite Halteteil können einen nutförmigen Abschnitt aufweisen, der derart angeordnet ist, dass das Kabel im geöffneten Zustand des Verschlusselementes mit einem ersten Abschnitt in der Nut aufgenommen ist und im geschlossenen Zustand mit einem zweiten Abschnitt in der Nut aufgenommen ist, der länger ist als der erste Abschnitt. Denkbar ist es beispielsweise, dass das Kabel im geöffneten Zustand des Verschlusselementes nicht oder nur mit einem Abschnitt in der Nut aufgenommen ist, der kürzer ist als der Abschnitt des Kabels, der im geschlossenen Zustand des Verschlusselementes in der Nut aufgenommen ist.

Der nutförmige Abschnitt des ersten Halteteils kann sich beispielsweise über einen Winkel von 180 ° erstrecken. Selbstverständlich sind auch andere Nutausführungen denkbar.

In weiterer Ausgestaltung der Erfindung weist das zweite Halteteil einen nutförmigen Abschnitt auf, der einen ersten Bereich und einen dazu parallel oder im wesentlichen parallel verlaufenden zweiten Bereich sowie einen U-förmigen Abschnitt aufweist, der den ersten mit dem zweiten Bereich verbindet. Dabei ist es denkbar, dass das Kabel im geöffneten Zustand des Verschlusselementes in einer Länge in dem ersten Bereich des nutförmigen Abschnitt aufgenommen ist, die geringer ist, als die Länge, in der das Kabel im geschlossenen Zustand in dem ersten Bereich des nutförmigen Abschnitts aufgenommen ist.

Vorzugsweise liegen der erste Bereich, der zweite Bereich sowie der U-förmige Abschnitt in einer Ebene.

Weiterhin kann vorgesehen sein, dass diese Ebene zu der Ebene senkrecht verläuft, in der die Nut des ersten Halteteils verläuft.

Die Halteteile können derart ausgeführt sein, dass im geschlossenen Zustand des Verschlußelementes der nutförmige Abschnitt des ersten Halteteils in seinem Endbereich mit dem Anfangsbereich des nutförmigen Abschnitt des zweiten Halteteils fluchtet.

Denkbar ist es, dass der Abschnitt des Kabels, der sich zwischen dem ersten und dem zweiten Halteteil erstreckt, im geöffneten Zustand des Verschlußelementes länger ist, als im geschlossenen Zustand des Verschlußelementes. Die Länge des freien Kabelabschnittes, der sich zwischen den Halteteilen erstreckt und der in keinem der nutförmigen Abschnitte aufgenommen ist, kann somit vom Öffnungs- bzw. Schließzustand des Verschlußelementes abhängen.

Weiterhin kann vorgesehen sein, dass das erste und das zweite Halteteil derart angeordnet sind, dass sie im geschlossenen Zustand des Verschlußelementes einen geringen Abstand zueinander aufweisen als im geöffneten Zustand des Verschlußelementes.

Die vorliegende Erfindung betrifft ferner ein Haushaltsgerät mit wenigstens einer Anordnung gemäß einem der Ansprüche 1 bis 11. Bei dem Haushaltsgerät handelt es sich beispielsweise um ein Kühl- und/oder Gefriergerät.

Weitere Einzelheiten und Vorteile der Erfindung werden anhand eines in der Zeichnung dargestellten Ausführungsbeispiels näher erläutert. Es zeigen:
Fig. 1, 2: eine Draufsicht sowie eine perspektivische Ansicht der Anordnung gemäß der Erfindung bei geöffnetem Verschlußelement und
Fig. 3, 4: eine Draufsicht sowie eine perspektivische Ansicht der Anordnung gemäß der Erfindung bei geschlossenem Verschlußelement.

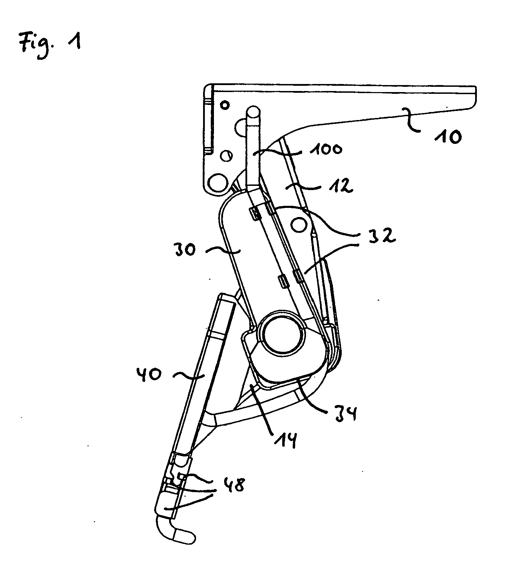

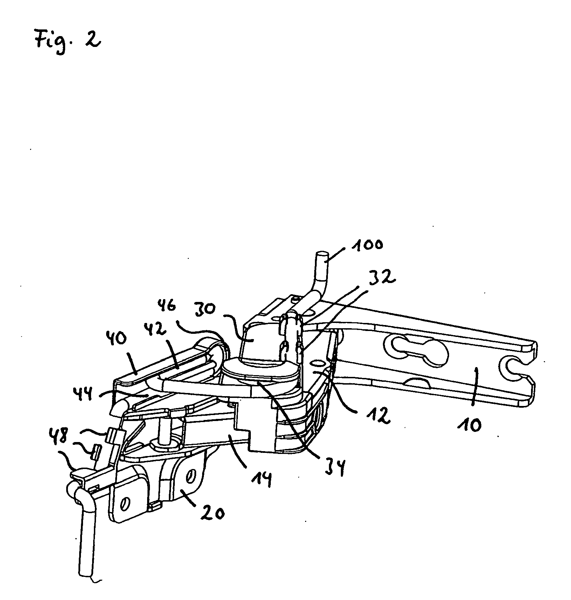

Figur 1 und 2 zeigen ein Mehrgelenkscharnier eines Kühlschrankes, mittels dessen die Kühlschranktür relativ zum Gerätekorpus verschwenkbar ist.

Das Mehrgelenkscharnier weist einen Korpusbeschlagskörper 10 auf, der im montierten Zustand des Scharniers mit dem Gerätekorpus in Verbindung steht und vorzugsweise mit diesem verschraubt ist. Das Bezugszeichen 20 kennzeichnet einen Türbeschlagskörper, der im montierten Zustand des Scharniers mit der Tür in Verbindung steht und vorzugsweise mit dieser verschraubt ist.

Mit den Bezugszeichen 12 und 14 sind zwei Hebel gekennzeichnet, von denen der Hebel 12 schwenkbar mit dem Korpusbeschlagskörper 10 und mit dem Hebel 14 und der Hebel 14 schwenkbar mit dem Hebel 12 und mit dem Türbeschlagskörper 20 in Verbindung steht.

Zur Führung des Kabels 100 über das Scharnier sind ein erstes Halteteil 30 und ein zweites Halteteil 40 vorgesehen. Das erste Halteteil 30 wird an eine im Hebel 12 angeordnete Bohrung mit Zentrierloch angeschraubt. Das zweite Halteteil 40 wird an den Türbeschlagskörper 20, d.h. an die Scharnierplatte, an die die Tür angeschraubt wird, aufgesteckt.

Sowohl das erste Halteteil 30 als auch das zweite Halteteil 40 bestehen aus Kunststoff, jedoch sind auch andere Materialien einsetzbar.

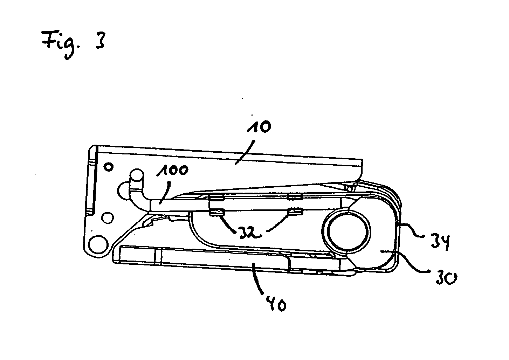

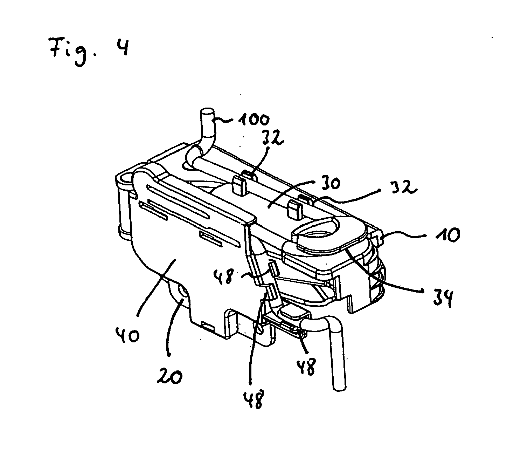

Das erste Halteteil 30 weist auf seiner Oberseite zwei Klammern 32 auf, zwischen denen das Kabel 100 fixiert ist. Das erste Halteteil 30 weist des Weiteren einen nutförmigen Abschnitt 34 auf, in den das durch die Klammern 32 fixierte Kabel 100 einläuft. Der nutförmige Abschnitt 34 erstreckt sich über einen Winkelbereich von 180 °. Wie dies aus den Figuren 3 und 4 hervorgeht, die die Anordnung in der Schließstellung des Verschlußelementes zeigen, liegt das Kabel 100 bei geschlossenem Scharnier in dem nutförmigen Abschnitt 34 über den genannten Winkelbereich von 180°. Wie dies aus den Figuren 1 und 2 hervorgeht, gilt dies nicht im geöffneten Zustand, in dem das Kabel nur im Anfangsbereich des nutförmigen Abschnittes 34 in diesem anliegt und sich davon ausgehend zu dem zweiten Halteteil 40 erstreckt.

Das zweite Halteteil 40 weist einen nutförmigen Abschnitt mit einem ersten Bereich 42 und einem zweiten Bereich 44 auf, die parallel zueinander verlaufen. Beide Bereiche stehen über den U-förmigen Abschnitt 46 miteinander in Verbindung. Ist das Scharnier bzw. das Verschlußelement geschlossen, liegt das Kabel über die gesamte Länge des ersten Bereichs 42, des U-förmigen Abschnitts 46 und des zweiten Bereichs 44 in dem nutförmigen Abschnitt des zweiten Halteteils 40. In diesem Fall liegen der Endbereich des nutförmigen Abschnitts 34 des ersten Halteteils 30 sowie der Anfangsbereich des nutförmigen Abschnitts bzw. des ersten Bereichs 42 benachbart zueinander, so dass nur ein vergleichsweise kurzer Abschnitt des Kabels 100 in keinem der nutförmigen Abschnitte 34, 42 geführt ist.

Wird das Verschlußelement und damit auch das Scharnier geöffnet, wird das Kabel bereichsweise aus dem nutförmigen Abschnitt 34 des ersten Halteteils 30 sowie aus dem ersten Bereich 42 des zweiten Halteteils 40 entfernt und erstreckt sich in gerader Linie zwischen beiden Halteteilen 30, 40, wie dies aus den Figuren 1 und 2 hervorgeht.

Im Anschluß an den zweiten Bereich 44 des zweiten Halteteils 40 wird das Kabel 100 mittels Klammern 48 des zweiten Halteteils 40 derart geführt, dass es zu einem in der Tür angeordneten Graphikdisplay oder dergleichen weiter verläuft.

Wie dies aus den Figuren hervorgeht, sind die Einzelteile der Kabelführung derart konstruiert, dass ein Einklemmen oder Quetschen des Kabels verhindert wird. Bei der Bewegung wird das Kabel um große Radien gebogen, wodurch ein Abknicken des Kabels verhindert wird. Wie ausgeführt, wird das Kabel beim Zusammenklappen bzw. Schließen des Scharniers an großzügigen Rundungen angelegt, die verhindern, dass das Kabel knickt oder in zu engen Radien bewegt wird. Die Anordnung gemäß der Erfindung ermöglicht es, eine große Zahl von Öffnungs- und Schließbewegungen durchzuführen, ohne das Kabel zu beschädigen.

Die Anordnung ist vorzugsweise derart ausgestaltet, dass sie auch nachträglich an ein bereits vorhandenes Scharnier angebracht werden kann. Vorzugsweise ist dabei vorgesehen, dass die Halteteile montiert werden können, ohne dass die Notwendigkeit besteht, das Scharnier zu demontieren.

## Patentansprüche

1. Scharnier mit einer Anordnung zur Führung eines Kabels von einem Korpus eines Möbelelementes oder Gerätes zu einem Verschlusselement, das relativ zu dem Korpus mittels des Scharniers verschwenkbar ist und mittels dessen der Korpus verschließbar ist, wobei das Scharnier einen Korpusbeschlagskörper (10) und einen Verschlusselementbeschlagskörper (20) sowie ein oder mehrerer zwichen Korpusbeschlagskörper (10) und Verschlusselementbeschlagskörper (20) schwenkbar angeordnete Verbindungselemente (12, 14) aufweist und die Anordnung wenigstens ein erstes Halteteil (30) und wenigstens ein zweites Halteteil (40) zur Führung des Kabels (100) aufweist, wobei die Halteteile (30, 40) Nuten oder Klammern aufweisen, mittels derer das Kabel (100) geführt ist und wobei das erste Halteteil (30) an einem ersten Teil (12) des Scharniers angeordnet ist
**, dadurch gekennzeichnet, dass** das zweite Halteteil (40) an einem zweiten Teil (20) des Scharniers angeordnet ist, das relativ zu dem ersten Teil (12) des Scharniers bewegbar ist und dass das erste (12) und/oder das zweite Teil des Scharniers durch das wenigstens eine Verbindungselement (12) gebildet wird.

2. Scharnier mit einer Anordnung nach Anspruch 1, **dadurch gekennzeichnet, dass** das erste (30) und/oder das zweite Halteteil (40) mit dem ersten (12) bzw. zweiten Teil (20) des Scharniers verschraubt oder durch eine Steckverbindung fixiert sind.

3. Scharnier mit einer Anordnung nach Anspruch 1 oder 2, **dadurch gekennzeichnet, dass** das erste oder das zweite Teil (20) des Scharniers durch den Korpusbeschlagskörper oder den den Verschlusselementbeschlagskörper (20) gebildet wird.

4. Scharnier mit einer Anordnung nach einem der vorhergehenden Ansprüche, **dadurch gekennzeichnet, dass** das erste Halteteil (30) sowie das zweite Halteteil (40) Kabelführungsbereiche aufweisen, die wenigstens abschnittsweise auf gleicher Höhe angeordnet sind.

5. Scharnier mit einer Anordnung nach einem der vorhergehenden Ansprüche, **dadurch gekennzeichnet, dass** das erste Halteteil (30) und/oder das zweite Halteteil (40) einen nutförmigen Abschnitt (34; 42, 44, 46) aufweist, der derart angeordnet ist, dass das Kabel (100) im geöffneten Zustand des Verschlusselementes mit einem ersten Abschnitt in dem nutförmigen Abschnitt (34; 42) aufgenommen ist und im geschlossenen Zustand mit einem zweiten Abschnitt in dem nutförmigen Abschnitt (34; 42) aufgenommen ist, der länger ist als der erste Abschnitt.

6. Scharnier mit einer Anordnung nach Anspruch 5, **dadurch gekennzeichnet, dass** sich der nutförmige Abschnitt (34) des ersten Halteteils (30) über einen Winkel von 180 ° erstreckt.

7. Scharnier mit einer Anordnung nach einem der vorhergehenden Ansprüche, **dadurch gekennzeichnet, dass** das zweite Halteteil (40) einen nutförmigen Abschnitt (42, 44, 46) aufweist, der einen ersten Bereich (42) und einen dazu parallel oder im wesentlichen parallel verlaufenden zweiten Bereich (46) sowie einen U-förmigen Abschnitt (44) aufweist, der den ersten (42) mit dem zweiten Bereich (46) verbindet.

8. Scharnier mit einer Anordnung nach Anspruch 7, **dadurch gekennzeichnet, dass** der erste Bereich (42), der zweite Bereich (44) sowie der U-förmige Abschnitt (46) in einer Ebene liegen.

9. Scharnier mit einer Anordnung nach Anspruch 8 und nach einem der Ansprüche 5 bis 7, **dadurch gekennzeichnet, dass** die Ebene senkrecht zu der Ebene liegt, in der der nutförmige Abschnitt (34) des ersten Halteteils (30) verläuft.

10. Scharnier mit einer Anordnung nach einem der Ansprüche 5 bis 9, **dadurch gekennzeichnet, dass** im geschlossenen Zustand des Verschlusselementes der nutförmige Abschnitt (34) des ersten Halteteils (30) in seinem Endbereich mit dem Anfangsbereich des nutförmigen Abschnitt (42) des zweiten Halteteils (40) fluchtet.

11. Scharnier mit einer Anordnung nach einem der vorhergehenden Ansprüche, **dadurch gekennzeichnet, dass** das erste (30) und das zweite Halteteil (40) derart angeordnet sind, dass sie im geschlossenen Zustand des Verschlusselementes einen geringen Abstand zueinander aufweisen als im geöffneten Zustand des Verschlusselementes.

12. Haushaltsgerät mit wenigstens einem Scharnier mit einer Anordnung gemäß einem der Ansprüche 1 bis 11.

13. Haushaltsgerät nach Anspruch 12, **dadurch gekennzeichnet, dass** es sich bei dem Haushaltsgerät um ein Kühl- und/oder Gefriergerät handelt.

## Claims

1. Hinge joint with an arrangement for conducting a cable from a body of a furniture element or device to a closing element that is pivotable relative to the body by means of the hinge joint and by means of which the body can be closed, wherein the hinge joint with the arrangement possesses at least one first retaining part (30) and at least one second retaining part (40) for conducting the cable (100), wherein the retaining parts (30, 40) have grooves or braces, by means of which the cable (100) is conducted, and wherein the first retaining part (30) is arranged at a first part (12) of the hinge joint and the second retaining part (40) is arranged at a second part (20) of the hinge joint that is moveable relative to the first part (12) of the hinge joint, **characterised in that** the hinge joint has a body fitting unit (10) and a closing element fitting unit (20) as well as one or more connecting elements (12, 14) arranged rotatably between the body fitting unit (10) and the closing element fitting unit (20) and, respectively, with the body fitting unit (10) and the closing element fitting unit (20), and **in that** the first (12) and/or second part of the hinge joint is formed by the at least one connecting element (12).

2. Hinge joint with an arrangement according to claim 1, **characterised in that** the first (30) and/or second retaining part (40) is screwed down with the first (12) or second part (20) of the hinge joint or fixed by a plug-in connection.

3. Hinge joint with an arrangement according to claim 1 or 2, **characterised in that** the first or second part (20) of the hinge joint is formed by the body fitting unit or the closing element fitting unit (20).

4. Hinge joint with an arrangement according to one of the preceding claims, **characterised in that** the first retaining part (30) and the second retaining part (40) have cable conducting regions, which are arranged at least in parts at the same height.

5. Hinge joint with an arrangement according to one of the preceding claims, **characterised in that** the first retaining part (30) and/or the second retaining part (40) has a groove-shaped section (34; 42, 44, 46), which is arranged in such a way that the cable (100) is received in the groove-shaped section (34; 42) when the closing element with a first section is opened and is received in the groove-shaped section (34; 42) when the closing element with a second section is in a closed state, which is longer than the first section.

6. Hinge joint with an arrangement according to claim 5, **characterised in that** the groove-shaped section (34) of the first retaining part (30) extends over an angle of 180°.

7. Hinge joint with an arrangement according to one of the preceding claims, **characterised in that** the second retaining part (40) has a groove-shaped section (42, 44, 46), which has a first region (42) and a second region (46) running parallel or essentially parallel thereto, as well as a U-shaped section (44), which connects the first (42) and the second region (46).

8. Hinge joint with an arrangement according to claim 7, **characterised in that** the first region (42), the second region (44) and the U-shaped section (46) lie in a plane.

9. Hinge joint with an arrangement according to claim 8 and according to one of claims 5 to 7, **characterised in that** the plane is perpendicular to the plane in which the groove-shaped section (34) of the first retaining part (30) runs.

10. Hinge joint with an arrangement according to one of claims 5 to 9, **characterised in that**, when the closing element is closed, the groove-shaped section (34) of the first retaining part (30) is aligned in its end region with the starting region of the groove-shaped section (42) of the second retaining part (40).

11. Hinge joint with an arrangement according to one of the preceding claims, **characterised in that** the first (30) and the second retaining section (40) are arranged in such a way that they have a small gap to one another when the closing element is closed than when the closing element is open.

12. Household device having at least one hinge joint with an arrangement according to one of claims 1 to 11.

13. Household device according to claim 12, **characterised in that** the household device is a cooling and/or freezing device.

## Revendications

1. Charnière comprenant un dispositif destiné à guider un câble depuis une structure d'un élément de meuble ou d'un appareil vers un élément de fermeture qui peut pivoter par rapport à la structure au moyen de la charnière et au moyen duquel la structure peut être fermée, étant entendu que la charnière comprend un corps de ferrure de la structure (10) et un corps de ferrure de l'élément de fermeture (20) ainsi qu'un ou plusieurs éléments de liaison (12, 14) agencés entre le corps de ferrure de la structure (10) et le corps de ferrure de l'élément de fermeture (20) et pivotant respectivement avec le corps de ferrure de la structure (10) et le corps de ferrure de l'élément de fermeture (20) et que le dispositif comprend au moins une première partie de support (30) et au moins une deuxième partie de support (40) afin de guider le câble (100), les parties de support (30, 40) comprenant des rainures ou des crochets au moyen desquels le câble (100) est guidé, et étant entendu que la première partie de support (30) est agencée sur une première partie (12) de la charnière, **caractérisée en ce que** la deuxième partie de support (40) est agencée sur une deuxième partie (20) de la charnière qui est mobile par rapport à la première partie (12) de la charnière et **en ce que** la première (12) et/ou la deuxième partie de la charnière est constituée par l'au moins un élément de liaison (12).

2. Charnière comprenant un dispositif selon la revendication 1, **caractérisée en ce que** la première (30) et/ou la deuxième (40) partie de support sont vissées ou fixées par un assemblage, respectivement, avec la première (12) et la deuxième (20) partie de la charnière.

3. Charnière comprenant un dispositif selon la revendication 1 ou 2, **caractérisée en ce que** la première ou la deuxième partie (20) de la charnière est constituée par le corps de ferrure de la structure ou le corps de ferrure de l'élément de fermeture (20).

4. Charnière comprenant un dispositif selon l'une des revendications précédentes, **caractérisée en ce que** la première partie de support (30) ainsi que la deuxième partie de support (40) comprennent des zones de guidage de câble qui sont agencées, au moins dans une section, à la même hauteur.

5. Charnière comprenant un dispositif selon l'une des revendications précédentes, **caractérisée en ce que** la première partie de support (30) et/ou la deuxième partie de support (40) comprend une section en forme de rainure (34 ; 42, 44, 46) qui est agencée de telle sorte que dans la position ouverte de l'élément de fermeture, le câble (100) est logé avec une première section dans la section en forme de rainure (34 ; 42), et dans la position fermée, il est logé avec une deuxième section dans la section en forme de rainure (34 ; 42), qui est plus longue que la première section.

6. Charnière comprenant un dispositif selon la revendication 5, **caractérisée en ce que** la section en forme de rainure (34) de la première partie de support (30) s'étend dans un angle de 180°.

7. Charnière comprenant un dispositif selon l'une des revendications précédentes, **caractérisée en ce que** la deuxième partie de support (40) comprend une section en forme de rainure (42, 44, 46) qui comprend une première zone (42) et une deuxième zone (46) parallèle ou sensiblement parallèle à celle-ci, ainsi qu'une section en forme de U (44) qui relie la première zone (42) avec la deuxième zone (46).

8. Charnière comprenant un dispositif selon la revendication 7, **caractérisée en ce que** la première zone (42), la deuxième zone (44) et la section en forme de U (46) se situent dans un plan.

9. Charnière comprenant un dispositif selon la revendication 8 et l'une des revendications 5 à 7, **caractérisée en ce que** le plan est perpendiculaire au plan dans lequel s'étend la section en forme de rainure (34) de la première partie de support (30).

10. Charnière comprenant un dispositif selon l'une des revendications 5 à 9, **caractérisée en ce que** dans la position fermée de l'élément de fermeture, la section en forme de rainure (34) de la première partie de support (30) est alignée dans sa zone de fin avec la zone de début de la section en forme de rainure (42) de la deuxième partie de support (40).

11. Charnière comprenant un dispositif selon l'une des revendications précédentes, **caractérisée en ce que** la première (30) et la deuxième (40) partie de support sont agencées de telle sorte que dans la position fermée de l'élément de fermeture, elles présentent un plus petit écart entre elles que dans la position ouverte de l'élément de fermeture.

12. Appareil ménager ayant au moins une charnière comprenant un dispositif selon l'une des revendications 1 à 11.

13. Appareil ménager selon la revendication 12, **caractérisé en ce que** cet appareil ménager est un appareil de réfrigération et/ou de congélation.
